# FASCICULE DE BREVET EUROPEEN

(11) **EP 1 599 138 B1**
(45) Date de publication et mention de la délivrance du brevet: **25.10.2006**
(21) Numéro de dépôt: 04712592.7
(22) Date de dépôt: 19.02.2004
(51) Int. Cl.: A61B 10/00

(54) **TROCART POUR BIOPSIE OSTEO-MEDULLAIRE**
TROKAR FÜR DIE OSTEOMEDULARE BIOPSIE
TROCHAR FOR OSTEO-MEDULLARY BIOPSY

(30) Priorité: 25.02.2003 FR 0302292
(43) Date de publication de la demande: 30.11.2005
(73) Titulaire: Slama, Borhane, 84000 Avignon (FR); Zerazhi, Hacène, 13570 Barbentane (FR); Verra, Yvan, 84450 Jonquerettes (FR)
(72) Inventeur: Slama, Borhane, 84000 Avignon (FR); Zerazhi, Hacène, 13570 Barbentane (FR); Verra, Yvan, 84450 Jonquerettes (FR)
(74) Mandataire: Dall'Olio, Giancarlo
(86) Numéro de dépôt international: PCT/FR2004/000391
(87) Numéro de publication internationale: WO 2004/075759

(56) Documents cités:
- EP-A- 1 252 863
- WO-A-99/62396
- GB-A- 1 063 653
- US-A- 2 541 542
- US-A- 4 785 826

## Description

La présente invention concerne un trocart pour biopsie ostéo-médullaire.

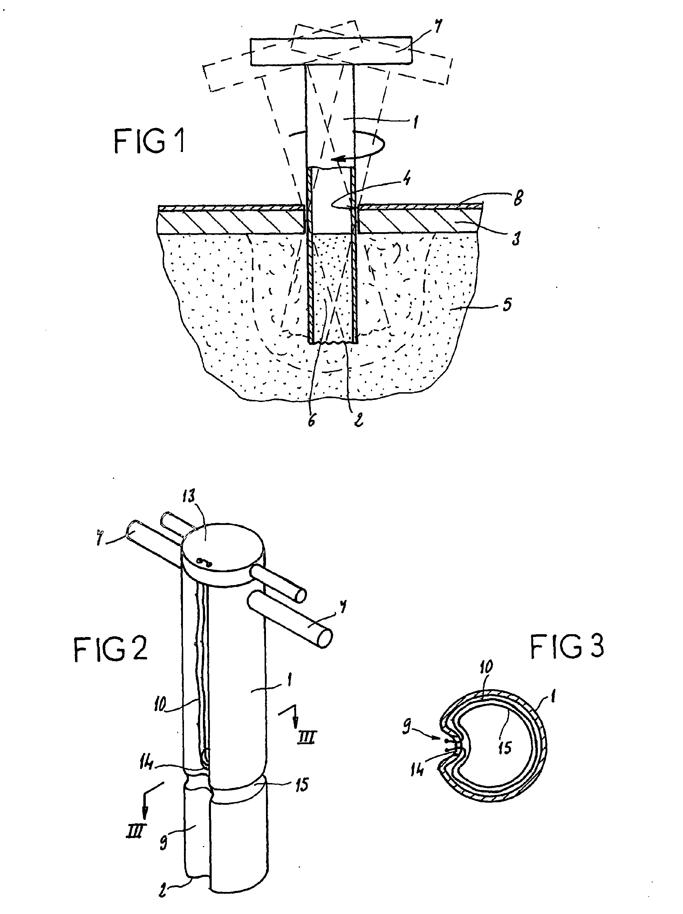

Les médecins font des biopsies ostéo-médullaires à des fins d'analyses permettant de détecter des anomalies de constitution afin dy remédier. Ils disposent d'un instrument appelé trocart, tel que représenté à la figure 1 du dessin ci-annexé, comprenant un tube 1 de quelques millimètres de diamètre dont une extrémité 2 est aiguisée pour pénétrer dans l'os 3 en le sciant circulairement afin d'effectuer un trou de forage 4 permettant d'accéder à la moelle osseuse 5 puis de découper un carottage 6 de moelle cylindrique, et dont l'autre extrémité est munie d'une poignée de maintien 7 permettant de manipuler en faisant tourner ledit tube afin de scier circulairement le périoste 8 (tissu richement innervé qui entoure l'os), puis l'os, puis la carotte de moelle.

Lorsque le carottage est fait et que le tronçon de moelle osseuse se trouve à l'intérieur du tube, le médecin doit détacher la carotte de moelle osseuse du reste de la moelle qui est résistante par sa constitution fibreuse. Pour ce faire, il est obligé de faire de grands mouvements circulaires et pendulaires (représentés en traits pointillés à la figure 1) avec la poignée afin d'essayer de détacher la carotte de moelle par arrachement ce qui est aléatoire (car la carotte se désagrège et n'est plus exploitable), extrêmement douloureux pour le patient, et enfin provoque des dégâts collatéraux sur le périoste, sur l'os et sur la moelle environnante. Cet acte médical barbare impressionne les médecins chargés de l'exécuter, et fait fuir les patients malgré les risques médicaux évidents dus au refus.

Le document WO 99/62396 décrit un trocart selon le préambule de la revendication 1.

Le but de la présente invention est de résoudre les inconvénients associés aux trocarts existants, tels que cités plus haut, et dans un but d'humanisation d'une part, et afin d'assurer le résultat du prélèvement d'autre part, de supprimer le mouvement circulaire et pendulaire d'arrachement de la carotte de moelle.

Les objectifs de la présente invention sont atteints avec un trocart pour biopsie ostéo-médullaire comprenant un tube dont une extrémité est aiguisée pour pénétrer dans un os par forage, et dont l'autre extrémité comporte une poignée permettant la manipulation dudit trocart.

Plus particulièrement, le trocart selon l'invention est caractérisé en ce que le tube délimite sur sa face extérieure, un canal longitudinal dont le fond est tourné vers l'intérieur du tube, et en ce qu'il comprend un fil à découper formant une boucle, dont les extrémités libres sont fixées à au moins une poignée de traction située du côté de la poignée du trocart, dont les brins sont disposés de manière coulissante dans le canal longitudinal, et dont la boucle traverse le tube par un orifice radial ménagé à distance de la poignée du trocart, des moyens étant prévus pour assurer le maintien de la boucle sous forme ouverte au contact de la paroi intérieure du tube, avant actionnement du fil.

Comme on l'aura compris, l'utilisation du trocart selon l'invention requiert seulement des mouvements circulaires - semblables à ceux effectués avec un tournevis - pour pouvoir réaliser le forage, avec une technique identique à celle utilisée avec les trocarts connus. Par contre, les mouvements circulaires et pendulaires d'arrachement de la moelle sont supprimés et remplacés par un geste de maintien du trocart une fois celui-ci en place dans l'os, accompagné d'une traction sur la poignée reliée au fil, de sorte que l'arrachement de moelle est ici remplacé par une découpe simple par coulissement et resserrement du fil autour de la carotte de moelle. Le geste chirurgical est donc beaucoup moins traumatisant physiquement pour le patient, et psychologiquement pour le praticien. Enfin, la qualité du prélèvement obtenu est assuré puisqu'il n'y a plus de risque de désagréger la carotte par des mouvements pendulaires du trocart.

De préférence, les moyens de maintien de la boucle du fil comprennent un bourrelet circulaire et dirigé vers l'intérieur du tube, disposé à proximité de l'orifice radial, du côté opposé à la poignée du trocart par rapport audit orifice. Un tel bourrelet permet avantageusement de maintenir la boucle du fil lorsque la carotte de moelle coulisse vers le haut à l'intérieur du tube.

D'autres moyens de maintien de la boucle du fil à découper pourraient être envisagés néanmoins, comme par exemple le collage provisoire de la boucle contre les parois intérieures du tube, à l'aide d'une colle compatible avec l'usage médical qui est fait du trocart, ou bien l'utilisation d'un fil métallique et de parois intérieures du tube aimantées de manière à retenir la boucle, ou encore l'utilisation d'une gorge circulaire disposée à la surface intérieure du tube, dans laquelle la boucle puisse être maintenue.

Selon une variante d'exécution préférée de l'invention, le trocart comprend en outre un tube disposé de manière concentrique à l'intérieur du tube de trocart et coulissant à l'intérieur de ce dernier, ledit tube coulissant étant fixé dans sa partie haute à la poignée de traction, sa hauteur étant telle qu'avant actionnement du fil, son extrémité inférieure vient affleurer le bourrelet de sorte que la boucle du fil à découper est bloquée entre la partie haute du bourrelet et la partie basse dudit tube coulissant.

Avantageusement, la poignée de traction est disposée au-dessus de la poignée du trocart, de manière à pouvoir s'encastrer dans cette dernière. Une telle disposition est très ergonomique puisque lorsque la poignée de traction est encastrée dans la poignée du trocart, l'ensemble formé est assez peu volumineux et permet donc une prise en main efficace, notamment pour effectuer les mouvements circulaires de forage de l'os.

De préférence, l'orifice radial de passage de la boucle de fil vers l'intérieur du tube comporte des bords arrondis et non tranchants, de manière à éviter le sectionnement du fil lors de son coulissement dans ledit orifice.

Selon une forme particulière d'exécution de la présente invention, le bourrelet est constitué par un redent formé par assemblage d'un second tube de trocart, de manière concentrique à l'intérieur du tube de trocart, par soudure, par collage, ou par traiement des surfaces en contact à l'émeri.

Enfin, on notera que le fil utilisé sera réalisé en un matériau souple, très fin, et suffisamment résistant pour trancher la moelle osseuse, par exemple un fil métallique, en matière synthétique, ou en matière naturelle, le matériau utilisé étant choisi de manière appropriée pour sa compatibilité avec l'usage médical.

Pour sa bonne compréhension, l'invention est à nouveau décrite ci-après en référence au dessin schématique annexé représentant, à titre d'exemple non limitatif, deux variantes d'exécution du trocart qu'elle concerne.
La figure 1 est une vue en coupe partielle d'un trocart de l'art antérieur ;
la figure 2 est une vue en perspective du trocart selon l'invention ;
la figure 3 est une vue en coupe selon III/III de figure 2 ;
la figure 4 est une vue en coupe du trocart selon l'invention après forage et avant découpe de la carotte ;
la figure 5 est une vue en coupe du trocart selon l'invention après forage et découpe de la carotte ;
la figure 6 est une vue similaire à figure 4 d'une variante d'exécution d'un trocart selon l'invention,
la figure 7 est une vue similaire à figure 5 d'une variante d'exécution d'un trocart selon l'invention.

Les figures 2, 4 et 5 représentent un trocart selon l'invention comportant un tube 1 dont l'extrémité 2 inférieure est acérée pour réaliser un trou de forage 4 dans le périoste 8, puis l'os 3 d'un patient, et afin d'atteindre la moelle osseuse 5.

Le tube 1 du trocart comporte à son extrémité supérieure une poignée de trocart (ou poignée de maintien) 7 qui permet au chirurgien d'utiliser le trocart comme un tournevis, c'est-à-dire avec des mouvements circulaires lorsqu'il réalise le forage, et d'autre part de maintenir le trocart en place dans l'os lorsqu'il réalise le prélèvement de moelle proprement dit.

Comme illustré à la figure 2, le tube 1 du trocart délimite sur sa face extérieure, un canal longitudinal 9 dont le fond est tourné vers l'intérieur du tube.

Un fil à découper 10 formant une boucle 12 est disposé le long du tube. Ses extrémités libres sont fixées à une poignée de traction 13 située au-dessus de la poignée de trocart 7. Les brins du fil sont disposés de manière coulissante au fond du canal longitudinal 9 de sorte qu'ils ne sont pas entraînés par le mouvement de rotation du trocart lorsque ce dernier est pivoté par le chirurgien pour réaliser le forage. La boucle 12 du fil à découper traverse la paroi du tube par un orifice radial 14 ménagé à distance de la poignée du trocart 7.

Comme illustré aux figures 2 et 3, un bourrelet circulaire 15 est aménagé à la face inférieure du tube, ledit bourrelet 15 étant dirigé vers l'intérieur du tube et disposé à proximité de l'orifice radial 14, du côté opposé à la poignée du trocart par rapport audit orifice.

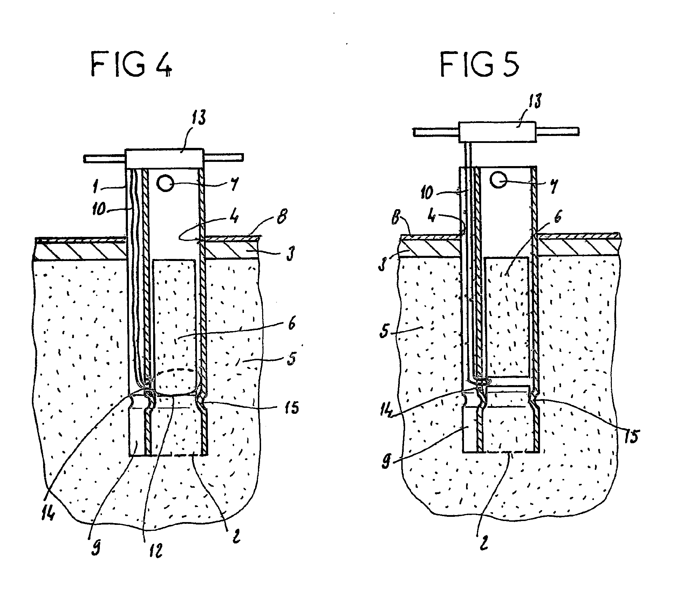

Comme montré à la figure 3, la boucle 12 du fil à découper repose normalement en appui sur le bourrelet, de sorte que lorsque la carotte de moelle remonte à l'intérieur du tube, comme montré à la figure 4, le fil n'est pas entraîné par ladite carotte, et la boucle reste ouverte.

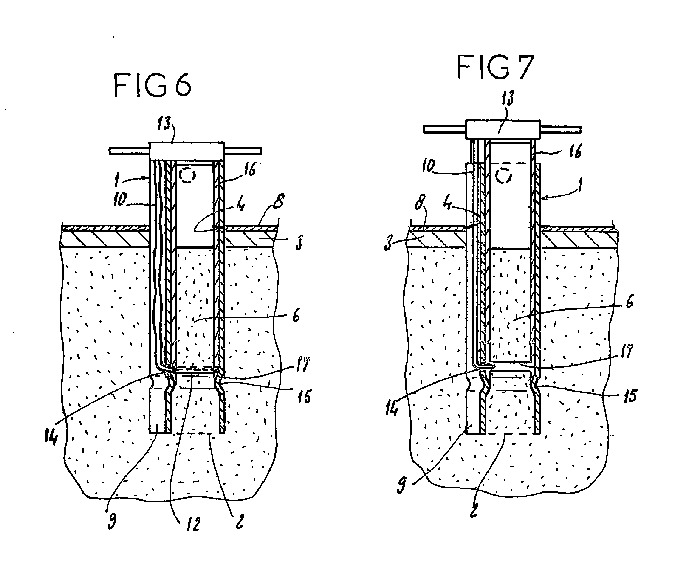

Comme illustré à la figure 2, la poignée de traction 13 est disposée au-dessus de la poignée du trocart, et est donc facilement accessible lorsque le praticien doit tirer dessus pour resserrer la boucle 12 autour de la carotte 6 et découper celle-ci, comme cela est illustré à la figure 5.

Avantageusement, l'orifice radial 14 permettant le passage de la boucle 12 de fil à l'intérieur du tube 1, est usiné de sorte que ses bords sont arrondis et donc non tranchants afin d'éviter le sectionnement du fil lors de son coulissement.

Après avoir perforé le périoste 8, puis l'os 3, puis découpé la moelle osseuse 5 par une action ressemblant à un vissage effectué avec doigté, il suffit de maintenir la poignée de trocart 7 et simultanément de tirer sur la poignée de traction 13 du fil à découper 10. Cette action très simple resserre la boucle 12 du fil à découper et sectionne par découpage la moelle osseuse 5 en séparant la carotte 6 de moelle osseuse du reste de la moelle 5.

Selon une variante d'exécution du trocart décrit ci-dessus, ledit trocart comprend en outre un tube 16 disposé de manière concentrique à l'intérieur du tube de trocart 1, et coulissant verticalement à l'intérieur de ce dernier, ledit tube coulissant 16 étant fixé dans sa partie haute à la poignée de traction 13 - éventuellement de manière détachable -, sa hauteur étant telle qu'avant actionnement du fil, son extrémité inférieure 17 vient affleurer le bourrelet 15.

De cette manière, la boucle 12 du fil à découper 10 est, avant actionnement du fil par usage de la poignée de traction, bloquée entre la partie haute du bourrelet 15 et la partie basse dudit tube coulissant 16.

Ceci permet de diminuer encore les risques que la boucle 12 du fil à découper ne soit emportée vers le haut par la carotte de moelle osseuse, lorsque l'on effectue le forage.

Un autre intérêt de ce tube coulissant 16 est qu'il permet de récupérer directement la carotte de moelle, qui ne reste pas bloquée à l'intérieur du tube de trocart 1.

Il va de soi que l'invention n'est pas limitée à la forme de réalisation décrite ci-dessus à titre d'exemple mais qu'elle en embrasse, au contraire, toutes les variantes de réalisation. Ainsi, la forme et le diamètre du tube pourront être adaptés aux différents types de biopsies à effectuer, en fonction de la nature de l'os à perforer (os long ou plat).

## Revendications

1. Trocart pour biopsie ostéo-médullaire comprenant un tube (1) dont une extrémité (2) est acérée pour pénétrer dans un os (3) par forage, et dont l'autre extrémité comporte une poignée (7) permettant la manipulation dudit trocart, comprenant un fil à découper (10) formant une boucle (12), dont les extrémités libres sont fixées à au moins une poignée de traction (13) située du côté de la poignée du trocart (7), et dont la boucle (12) traverse le tube par un orifice radial (14) ménagé à distance de la poignée du trocart, des moyens (15) étant prévus pour assurer le maintien de la boucle sous forme ouverte au contact de la paroi intérieure du tube, avant actionnement du fil, **caractérisé en ce que** le tube (1) délimite sur sa face extérieure, un canal longitudinal (9) dont le fond est tourné vers l'intérieur du tube, et **en ce que** les brins sont disposés de manière coulissante dans le canal longitudinal (9).

2. Trocart selon la revendication 1, **caractérisé en ce que** les moyens de maintien (15) de la boucle (12) du fil comprennent un bourrelet (15) circulaire et dirigé vers l'intérieur du tube (1), disposé à proximité de l'orifice radial (14), du côté opposé à la poignée du trocart (7) par rapport audit orifice (14).

3. Trocart selon la revendication 2, **caractérisé en ce qu'**il comprend en outre un tube (16) disposé de manière concentrique à l'intérieur du tube de trocart (1) et coulissant à l'intérieur de ce dernier, ledit tube coulissant (16) étant fixé dans sa partie haute à la poignée de traction (13), sa hauteur étant telle qu'avant actionnement du fil, son extrémité inférieure (17) vient affleurer le bourrelet (15) de sorte que la boucle (12) du fil à découper (10) est bloquée entre la partie haute du bourrelet (15) et la partie basse dudit tube coulissant (16).

4. Trocart selon l'une quelconque des revendications 1 à 3, **caractérisé en ce que** la poignée de traction (13) est disposée au-dessus de la poignée du trocart (7), de manière à pouvoir s'encastrer dans cette dernière.

5. Trocart selon l'une quelconque des revendications 1 à 4, **caractérisé en ce que** l'orifice radial (14) de passage de la boucle (12) de fil vers l'intérieur du tube (1) comporte des bords arrondis et non tranchants.

6. Trocart selon la revendication 2, **caractérisé en ce .que** le bourrelet (15) est constitué par un redent formé par assemblage d'un second tube de trocart de manière concentrique à l'intérieur du tube de trocart (1).

## Claims

1. Trocar for osteo-medullary biopsy comprising a tube (1), one end (2) of which is sharp in order to penetrate a bone (3) by drilling, and the other end of which comprises a handle (7) permitting manipulation of the said trocar, the said trocar comprising a cutting wire (10) forming a loop (12), the free ends of which are fixed to at least one traction handle (13) situated on the same side as the handle of the trocar (7), and the loop (12) of which passes through the tube through a radial orifice (14) provided at a distance from the handle of the trocar, means (15) being provided to ensure the holding of the loop in open form in contact with the internal wall of the tube, before actuation of the wire, **characterised in that** the tube (1) delimits, on its external face, a longitudinal channel (9) whose bottom is turned towards the inside of the tube, and **in that** the lengths are disposed so as to slide in the longitudinal channel (9).

2. Trocar according to claim 1, **characterised in that** the means (15) of holding the loop (12) of the wire comprise a circular rim (15) directed towards the inside of the tube (1), disposed close to the radial orifice (14), on the opposite side to the handle of the trocar (7) with respect to the said orifice (14).

3. Trocar according to claim 2, **characterised in that** it also comprises a tube (16) disposed concentrically inside the trocar tube (1) and sliding inside the latter, the said sliding tube (16) being fixed in its top part to the traction handle (13), its height being such that, before actuation of the wire, its bottom end (17) comes flush with the rim (15) so that the loop (12) of the cutting wire (10) is locked between the top part of the rim (15) and the bottom part of the said sliding tube (16).

4. Trocar according to any one of claims 1 to 3, **characterised in that** the traction handle (13) is disposed above the handle of the trocar (7), so as to be able to fit in the latter.

5. Trocar according to any one of claims 1 to 4, **characterised in that** the radial orifice (14) for passage of the loop (12) of wire towards the inside of the tube (1) comprises rounded non-cutting edges.

6. Trocar according to claim 2, **characterised in that** the rim (15) consists of a step formed by the assembly of a second trocar tube concentrically inside the trocar tube (1).

## Patentansprüche

1. Punktionsnadel für die Knochenmarksbiopsie, mit einem Rohr (1), dessen eines Ende (2) geschärft ist, um mittels Bohren in einen Knochen (3) einzudringen, und dessen anderes Ende einen Griff (7) aufweist, der die Handhabung der Punktionsnadel gestattet, wobei die Punktionsnadel einen eine Schlinge (12) bildenden Schneidedraht (10) umfasst, dessen freie Enden an mindestens einem auf der Seite des Griffes der Punktionsnadel (7) angeordneten Zuggriff (13) befestigt sind, und dessen Schlinge (12) das Rohr durch eine vom Griff der Punktionsnadel entfernt ausgebildete Radialöffnung (14) durchquert, wobei Mittel (15) vorgesehen sind, um den Halt der Schlinge in offener Form beim Kontakt mit der Innenwand des Rohrs vor Betätigung des Drahtes sicherzustellen, **dadurch gekennzeichnet, dass** das Rohr (1) an seiner Außenseite einen Längskanal (9) begrenzt, dessen Boden dem Inneren des Rohrs zugewandt ist, und dass die Stränge gleitend im Längskanal (9) angeordnet sind.

2. Punktionsnadel nach Anspruch 1, **dadurch gekennzeichnet, dass** die Haltemittel (15) für die Schlinge (12) des Drahtes einen kreisförmigen, zum Inneren des Rohrs (1) gerichteten Wulst (15) aufweisen, der nahe der Radialöffnung (14), auf der relativ zur Öffnung (14) dem Griff der Punktionsnadel (7) gegenüberliegenden Seite angeordnet ist.

3. Punktionsnadel nach Anspruch 2, **dadurch gekennzeichnet, dass** sie ferner ein Rohr (16) aufweist, das konzentrisch innerhalb des Rohrs der Punktionsnadel (1) und in diesem letzteren gleitend angeordnet ist, wobei das gleitende Rohr (16) mit seinem oberen Teil am Zuggriff (13) befestigt ist, und seine Höhe dergestalt ist, dass sein unteres Ende (17) vor Betätigung des Drahtes am Wulst (15) so zur Anlage kommt, dass die Schlinge (12) des Schneidedrahtes (10) zwischen dem oberen Teil des Wulstes (15) und dem unteren Teil des gleitenden Rohrs (16) arretiert ist.

4. Punktionsnadel nach einem der Ansprüche 1 bis 3, **dadurch gekennzeichnet, dass** der Zuggriff (13) über dem Griff der Punktionsnadel (7) so angeordnet ist, dass er in diesem letzteren versenkbar ist.

5. Punktionsnadel nach einem der Ansprüche 1 bis 4, **dadurch gekennzeichnet, dass** die Radialöffnung (14) zum Hindurchführen der Drahtschlinge (12) in das Innere des Rohrs (1) abgerundete, unscharfe Kanten aufweist.

6. Punktionsnadel nach Anspruch 2, **dadurch gekennzeichnet, dass** der Wulst (15) aus einem Absatz besteht, der durch konzentrisches Anbringen eines zweiten Punktionsnadel-Rohrs im Inneren des Rohrs der Punktionsnadel (1) gebildet ist.
